# EUROPÄISCHE PATENTSCHRIFT

(11) **EP 0 412 348 B1**
(45) Veröffentlichungstag und Bekanntmachung des Hinweises auf die Patenterteilung: **14.06.1995**
(21) Anmeldenummer: 90114063.2
(22) Anmeldetag: 23.07.1990
(51) Int. Cl.: G01N 3/04

(54) **Biegenmomentfreie Einspannvorrichtung für Prüfkörper oder Proben, insbesondere Keramikproben**
Specimen clamping device exerting no bending moment, in particular for ceramic test piece
Dispositif de fixation sans moment de flexion pour éprouvettes ou échantillons, en particulier échantillons de céramique

(30) Priorität: 09.08.1989 DE 3926308
(43) Veröffentlichungstag der Anmeldung: 13.02.1991
(73) Patentinhaber: CARL SCHENCK AG, D-64273 Darmstadt (DE)
(72) Erfinder: Pohl, Andreas, Ing.-grad., D-6114 Gross-Umstadt (DE)
(74) Vertreter: Brandt, Ernst-Ulrich, Dipl.-Phys., Dipl.-Ing.

(56) Entgegenhaltungen:
- GB-A- 990 621
- JOURNAL OF SCIENTIFIC INSTRUMENTS, Band 44, Mai 1967, Seiten 389-390; J.R.HOLLAND: "Self-aligning grip system and tensile specimens for Instron machines"
- PATENT ABSTRACTS OF JAPAN, Band 4, Nr. 62 (P-10)[544], 9. Mai 1980 ; & JP-A-55 31 974

## Beschreibung

Die Erfindung betrifft eine biegemomentfreie Einspannvorrichtung für Prüfkörper oder Proben, insbesondere Keramikproben, zur Zug- und/oder Druckbelastung der Probe, mit Aufnahmeelementen zur formschlüssigen Aufnahme der Probenenden, wobei die Aufnahmeelemente kugelige Außenflächen aufweisen, die in kugeligen Gegenflächen aufgenommen sind.

Bei der Zug- und/oder Druckprüfung von Prüfkörpern oder Proben in Prüfmaschinen soll die Probe möglichst genau definierten Belastungen ausgesetzt werden. So sollen beim Einspannen und bei Belastung der Probe keine unerwünschten Biegebeanspruchungen auf die Probe aufgebracht werden. Solche Beanspruchungen können z. B. durch Verkanten der Probe oder Versatz der Einspannköpfe, durch Fertigungsungenauigkeiten an den Einspannstellen der Probe oder ähnliche Faktoren auftreten. Dies trifft insbesondere für Keramikproben zu, die besonders empfindlich gegenüber Biegebeanspruchungen sind.

Es sind eine Reihe von Vorrichtungen bekannt, mit denen versucht wurde, biegemomentfreie Einspannungen für Prüfkörper oder Proben zu schaffen. Hierbei wird die Probe an sphärischen Flächen abgestützt bzw. gehalten. Die Vorrichtungen sind jedoch für Zug- und/oder Druckprüfung z. B. von Keramikproben nicht verwendbar. Entweder erlauben die bekannten Vorrichtungen nur eine Zugprüfung (DE-PS 671 954, DE-AS 23 06 393) oder sie erfordern besondere Spanneinrichtungen zum Einspannen der Probenenden oder Probenköpfe, da die Prüfkraft durch Reib- bzw. Kraftschluß über Aufnahmeelemente auf die Probe bzw. die Probenenden übertragen wird (DE-PS 2 028 030).

Aus der JP 55-31974 ist eine biegemomentfreie Einspannung für Proben oder Prüfkörper bekannt, wobei die Probenenden einen Gewindeabschnitt aufweisen und in Gewindebohrungen von kugeligen Aufnahmeelementen eingeschraubt werden. Die kugeligen Aufnahmeelemente sind in entsprechenden Kugelschalen gelagert.

Es ist Aufgabe der vorliegenden Erfindung, die Nachteile des Standes der Technik zu vermeiden und eine möglichst einfache und leicht zu betätigende biegemomentfreie Einspannvorrichtung, insbesondere für Keramikproben zu schaffen. Diese Aufgabe wird durch die in den Patentansprüchen angegebenen Merkmale gelöst.

Durch die Verwendung von Kugeln, die in passenden Löchern oder Querbohrungen an den Probenenden einsetzbar sind, ergibt sich eine einfache, leicht herzustellende und leicht zu betätigende Einspannvorrichtung, die insbesondere für sehr spröde Proben geeignet ist. Die kugelige Lagerung der Probenenden verhindert die Entstehung von Biegemomenten an der Probe beim Einspannvorgang und bei der nachfolgenden Belastung der Probe, da sich die Probenenden weitgehend zwangsfrei aufeinander einstellen können. Die Kugeln können als Stahl-, Keramik-, Hartmetall- oder Verbundwerkstoffkugeln ausgebildet sein, wobei z. B. auch handelsübliche Lagerkugeln verwendbar sind.

Eine zweckmäßige Ausgestaltung ergibt sich, wenn die Kugeln in Kugelschalen aufgenommen werden, die über je einen Haltering oder dgl. gegen die Kugeln spielfrei anlegbar sind. Eine schnelle und sichere Verbindung zwischen Kugeln und Kugelschalen ist dadurch herstellbar, daß die Kugelschalen konische oder keilförmige Außenflächen und die Halteringe entsprechend ausgebildete Gegen- bzw. Innenflächen aufweisen. Zweckmäßigerweise sind die Kugelschalen in oder an Kraftübertragungselementen angeordnet, die mit weiteren Elementen der Einspannvorrichtung oder mit der Prüfmaschine verbindbar sind. Die gegenseitige Einstellbarkeit der Probenenden, insbesondere unter Belastung, wird noch verbessert, wenn die Kugeln und/oder die Kugelschalen eine Beschichtung mit niedrigem Reibbeiwert aufweisen und/oder zwischen Kugeln und Kugelschalen ein Schmiermittel eingebracht ist. Zur Aufnahme von Flachproben können die Kugeln mit ringförmigen Schleifflächen am Kugelumfang versehen werden. Die Enden der Flachproben werden mit Löchern oder Querbohrungen hergestellt, die passend zu den Schleifflächen an den Kugeln ausgebildet sind. Die ringförmigen Schleifflächen und Löcher oder Querbohrungen an den Probenenden können hierbei zylindrisch oder konisch (kegelstumpfförmig) ausgeführt sein.

Eine vorteilhafte Ausgestaltung ergibt sich, wenn bei konischer bzw. kegelstumpfförmiger Ausbildung der Schleifflächen bzw. der Bohrungen an den Probenenden die Konizität bzw. der Kegelwinkel der Schleifflächen und der Bohrungen so klein gewählt wird, daß an der Schleif- bzw. Auflagefläche bei Zug- oder Druckbelastung der Probe Selbsthemmung zwischen der Probe und der Kugel in Bezug auf Relativbewegungen quer zur Belastungsrichtung eintritt. Hierdurch ist auf einfache Weise eine spielfreie Verbindung zwischen Probe und Kugel herstellbar. Eine solche Verbindung zwischen Probe und Einspannvorrichtung ist erforderlich, wenn Wechselbelastungen (Zug-Druck) auf die Probe aufgebracht werden sollen.

Die Erfindung wird nachstehend an Ausführungsbeispielen in den Zeichnungen dargestellt und in der Beschreibung näher erläutert.

Es zeigen:
- Fig. 1: Einspannvorrichtung für eine gelochte Flachprobe (Prüfung auf Zug)
- Fig. 2: Einspannvorrichtung für eine Rundprobe (Prüfung auf Zug und/oder Druck)
- Fig. 3: Kugel und gelochte Flachprobe mit konischer bzw. kegelstumpfförmiger Ausbildung der Schleifflächen

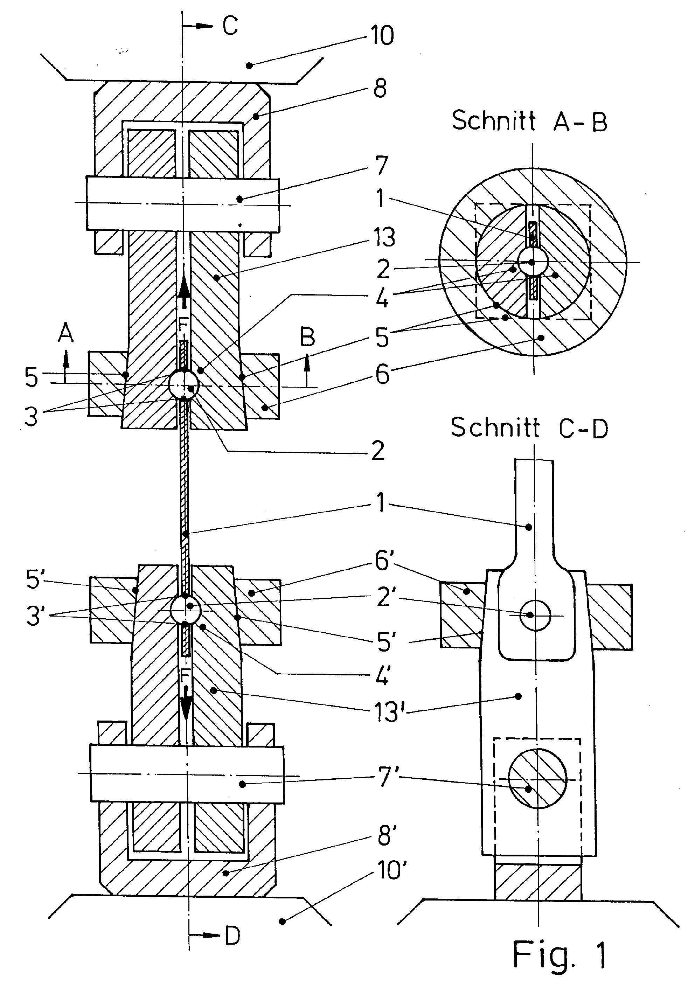

In Fig. 1 ist eine Flachprobe 1 an beiden Enden, wie dargestellt, mit Löchern oder Bohrungen 9 (s. a. Fig. 4) versehen, in denen Kugeln 2, 2′ angeordnet sind. Die Kugeln können aus bekannten Werkstoffen bestehen, z. B. Stahl, Keramik, Hartmetall oder Verbundwerkstoffen. Hierbei sind auch handelsübliche Lagerkugeln verwendbar. Die Durchmesser der Kugeln und die Lochdurchmesser der Proben sind aufeinander abgestimmt. Die Kugel- bzw. Lochdurchmesser werden möglichst klein gewählt. Die Kugeln 2, 2′ weisen an ihrem Umfang ringförmige bzw. zylindrische Schleifflächen 3, 3′ für die Aufnahme der Flachproben auf. Die Bohrungen 9 der Flachproben sind ebenfalls zylindrisch ausgeführt, so daß die Proben flächig auf den Kugeln aufliegen. Es kann jedoch auch eine linienförmige Auflage zwischen den Kugeln 2, 2′ und der Flachprobe 1 vorgesehen werden, wobei z.B. die Kugeln nicht angeschliffen sind und die Flachproben zylindrische Bohrungen aufweisen.

Die Kugeln 2, 2′ sind jeweils in Kugelschalen 4, 4′ aufgenommen die keilförmige oder konische Außenflächen 5, 5′ (Schnitt A-B) aufweisen. Über entsprechend ausgebildete Halteringe 6, 6′, die im Ausführungsbeispiel rund sind (s. Schnitt A-B) und zu den konischen Außenflächen 5, 5′ passende Gegen- bzw. Innenflächen aufweisen, sind die Kugelschalen 4, 4′ gegen die Kugeln 2, 2′ anlegbar. Hierzu werden die Halteringe über den jeweiligen Konus so verschoben, daß sich eine spielfreie Verbindung zwischen Kugeln und Kugelschalen ergibt.

Der Konus an den Außenflächen 5 und 5′ der oberen und unteren Kugelschalen 4 und 4′ kann, wie dargestellt, unterschiedlich ausgebildet sein (5′ zur Probe hin verlaufend, 5 von der Probe weg verlaufend), so daß bei einer senkrechten Einspannanordnung auch der obere Haltering 6 selbsttätig in der Endlage fixiert ist. Der Konus 5 kann jedoch auch wie Konus 5′ ausgeführt werden. In diesem Fall muß der Haltering 6 in seiner Betriebslage durch Federelemente, Stifte oder dgl. gesichert werden.

Die Halteringe 6, 6′ können z. B. bei keilförmigen Außenflächen 5, 5′ (im Schnitt A-B gestrichelt angedeutet) auch eine rechteckige oder andere geeignete Form aufweisen oder in anderer Weise ausgebildet sein, z. B. in Form von Haltebügeln.

Die Kugeln und/oder die Kugelschalen können mit einer Beschichtung, z. B. aus einem Kunststoff, Metallkarbiden oder Nitriten oder dgl. mit niedrigem Reibbeiwert, versehen sein. Zwischen Kugeln und Kugelschalen kann zusätzlich ein Schmiermittel eingebracht werden. Auf diese Weise wird die Beweglichkeit zwischen Kugeln und Kugelschalen erhöht bzw. die Reibung weiter herabgesetzt, so daß auch unter Belastung eine Relativbewegung zwischen Kugeln und Kugelschalen möglich ist.

Die Kugelschalen 4, 4′ sind in oder an Kraftübertragungselementen 13, 13′ angeordnet, die in Bolzen 7, 7′ gelagert sind. Die Bolzen sind über Lagerböcke 8, 8′ oder in anderer geeigneter Weise mit einer Prüfmaschine 10, 10′ (Traverse oder Antrieb der Prüfmaschine) zur Einleitung der Zugkraft F verbunden. Die Verbindung der Kugelschalen 4, 4′ bzw. der Kraftübertragungselemente 13, 13′ mit der Prüfmaschine kann auch anders als dargestellt ausgeführt sein. Die in Fig. 1 dargestellte Anordnung ist für die Prüfung auf Zug geeignet.

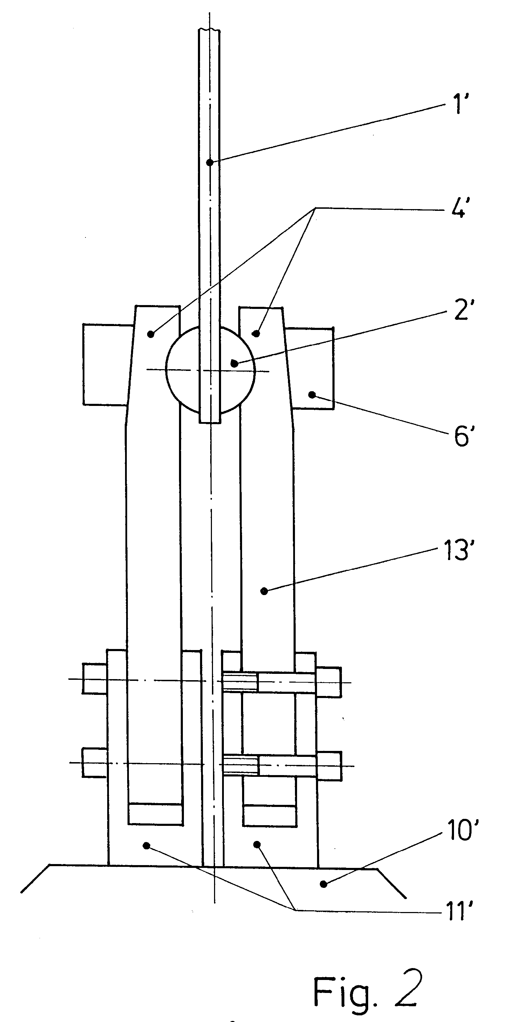

In Fig. 2 ist eine Anordnung (im Ausschnitt) dargestellt, die sowohl für Zug- als auch für Druckprüfungen geeignet ist. Hierbei werden die Kugelschalen 4, 4′ bzw. die Kraftübertragungselemente 13, 13′ in Klemmelementen 11′ form- oder kraftschlüssig, z. B. durch Verschrauben, aufgenommen. Die Klemmelemente 11′ sind starr mit der Prüfmaschine 10′ verbunden. Auf diese Weise können sowohl Zug- als auch Druckprüfungen an der Probe 1 vorgenommen werden. Die übrige Anordnung entspricht Fig. 1.

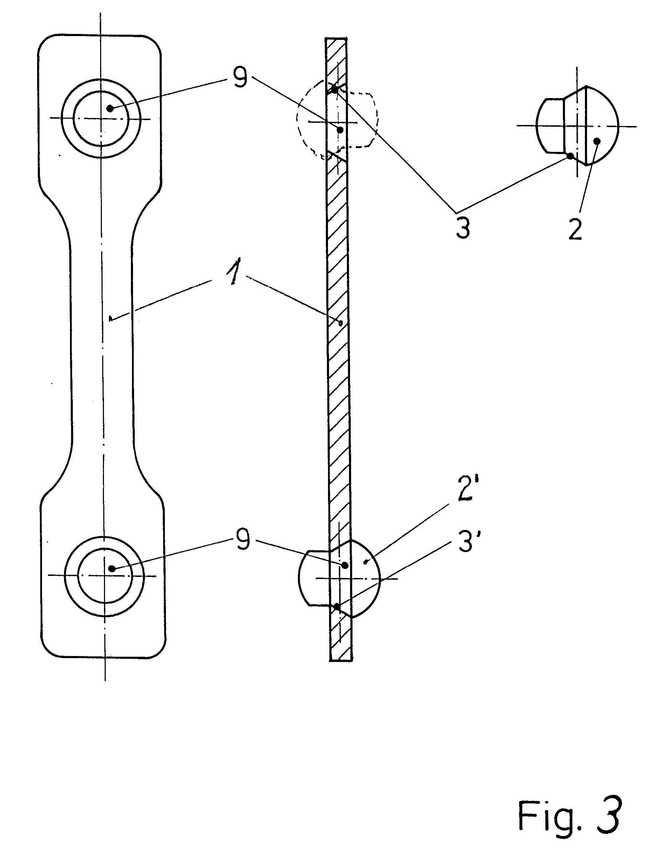

Für Zug- und Druckprüfungen können auch gelochte Proben verwendet werden. Die Schleifflächen 3, 3′ an den Kugeln 2, 2′ sowie die Bohrungen 9 an den Probenenden werden hierbei, wie in Fig. 3 dargestellt, konisch bzw. kegelstumpfförmig ausgeführt. Der Kegel- bzw. Keilwinkel wird so klein gewählt, daß Selbsthemmung zwischen Kugel 2, 2′ und Probe 1 bei Relativbewegungen quer zur Belastungsrichtung eintritt. Auf diese Weise wird eine spielfreie Verbindung zwischen Kugel und Probe hergestellt, wie sie für Wechselbelastungen (Zug-Druck) erforderlich ist. Die Proben können z. B. durch Kleben, auch punktweise, an den Kugeln fixiert werden. Eine Fixierung der Probe 1 an den Kugeln 2, 2′ ist auch dadurch möglich, daß die Kugeln so in die Kugelschalen 4, 4′ eingesetzt sind, daß die Kegelwinkel an den beiden Enden der Probe 1 gegensinnig verlaufen, wie in Fig. 3 gestrichelt dargestellt. Die Querbohrungen an der Probe werden entsprechend ausgebildet.

## Patentansprüche

1. Biegemomentfreie Einspannvorrichtung für Prüfkörper oder Proben, insbesondere Keramikproben, zur Zug- und/oder Druckbelastung der Probe, mit Aufnahmeelementen zur formschlüssigen Aufnahme der Probenenden, wobei die Aufnahmeelemente kugelige Außenflächen aufweisen, die in kugeligen Gegenflächen aufgenommen sind, dadurch gekennzeichnet, daß die Aufnahmeelemente als Kugeln (2, 2′) ausgebildet sind, die in passenden Löchern oder Querbohrungen (9) an den Probenenden einsetzbar sind.

2. Biegemomentfreie Einspannvorrichtung nach Anspruch 1, dadurch gekennzeichnet, daß die Kugeln (2, 2′) als Stahl-, Keramik-, Hartmetall- oder Verbund-Werkstoffkugeln ausgebildet sind.

3. Biegemomentfreie Einspannvorrichtung nach Anspruch 1 oder 2, dadurch gekennzeichnet, daß die Kugeln (2, 2′) in Kugelschalen (4, 4′) aufgenommen sind, die über je einen Haltering (6, 6′) oder dgl. gegen die Kugeln (2, 2′) spielfrei anlegbar sind.

4. Biegemomentfreie Einspannvorrichtung nach einem der vorhergehenden Ansprüchen, dadurch gekennzeichnet, daß die Kugelschalen (4, 4′) konische oder keilförmige Außenflächen (5, 5′) und die Halteringe (6, 6′) zu den Außenflächen (5, 5′) passende Gegenflächen aufweisen.

5. Biegemomentfreie Einspannvorrichtung nach einem der vorhergehenden Ansprüche, dadurch gekennzeichnet, daß die Kugeln (2, 2′) und/oder die Kugelschalen (4, 4′) eine Beschichtung mit niedrigem Reibbeiwert aufweisen.

6. Biegemomentfreie Einspannvorrichtung nach einem der vorhergehenden Ansprüche, dadurch gekennzeichnet, daß zwischen Kugeln (2, 2′) und Kugelschalen (4, 4′) ein Schmiermittel eingebracht ist.

7. Biegemomentfreie Einspannvorrichtung nach einem der vorhergehenden Ansprüche, dadurch gekennzeichnet, daß die Kugeln (2, 2′) für die Aufnahme von Flachproben (1) jeweils eine ringförmige Schleiffläche (3, 3′) am Kugelumfang aufweisen.

8. Biegemomentfreie Einspannvorrichtung nach Anspruch 7, dadurch gekennzeichnet, daß die Schleiffläche (3, 3′) zylindrisch oder konisch (kegelstumpfförmig) ausgebildet ist und die Löcher bzw. Querbohrungen (9) in den Probenenden jeweils an die Ausbildung der Schleifflächen (3, 3′) angepaßt sind.

9. Biegemomentfreie Einspannvorrichtung nach Anspruch 8, dadurch gekennzeichnet, daß bei konischer (kegelstumpfförmiger) Ausbildung der Schleiffläche (3, 3′) die Konizität bzw. der Kegelwinkel so klein gewählt wird, daß an der Schleif- bzw. Auflagefläche bei Zug- oder Druckbelastung der Probe (1) Selbsthemmung zwischen der Probe (1) und der Kugel (2, 2′) in Bezug auf Relativbewegungen quer zur Belastungsrichtung (F) eintritt.

10. Biegemomentfreie Einspannvorrichtung nach Anspruch 8 oder 9, dadurch gekennzeichnet, daß bei konischer Ausbildung der Schleifflächen (3, 3′) die Kugeln (2, 2′) an den Probenenden so in die Kugelschalen (4, 4′) eingesetzt sind, daß die Kegelwinkel gegensinnig zueinander verlaufen.

## Claims

1. Bending-moment-free clamping apparatus for test specimens or samples, in particular ceramic samples, for tensile and/or compression loading of the sample, having receiving elements for positively receiving the sample ends, the receiving elements having spherical outer surfaces which are accommodated in spherical countersurfaces, characterized in that the receiving elements take the form of balls (2, 2′) which are insertable into matching holes or transverse bores (9) at the sample ends.

2. Bending-moment-free clamping apparatus according to claim 1, characterized in that the balls (2, 2′) take the form of steel, ceramic, hard metal or composite material balls.

3. Bending-moment-free clamping apparatus according to claim 1 or 2, characterized in that the balls (2, 2′) are accommodated in spherical shells (4, 4′) which may be applied in each case by means of a retaining ring (6, 6′) or the like without play against the balls (2, 2′).

4. Bending-moment-free clamping apparatus according to one of the preceding claims, characterized in that the spherical shells (4, 4′) have conical or wedge-shaped outer surfaces (5, 5′) and the retaining rings (6, 6′) have countersurfaces which match the outer surfaces (5, 5′).

5. Bending-moment-free clamping apparatus according to one of the preceding claims, characterized in that the balls (2, 2′) and/or the spherical shells (4, 4′) have a coating with a low coefficient of friction.

6. Bending-moment-free clamping apparatus according to one of the preceding claims, characterized in that a lubricant is introduced between the balls (2, 2′) and the spherical shells (4, 4′).

7. Bending-moment-free clamping apparatus according to one of the preceding claims, characterized in that the balls (2, 2′) each have an annular facet (3, 3′) at the ball periphery for receiving flat samples (1).

8. Bending-moment-free clamping apparatus according to claim 7, characterized in that the facet (3, 3′) is cylindrical or conical (truncated-cone-shaped) and the holes or transverse bores (9) in the sample ends are in each case adapted to the construction of the facets (3, 3′).

9. Bending-moment-free clamping apparatus according to claim 8, characterized in that, given a conical (truncated-cone-shaped) construction of the facet (3, 3′), the amount of taper or the cone angle is selected low enough to produce at the facet or bearing surface, upon tensile or compression loading of the sample (1), self-locking between the sample (1) and the ball (2, 2′) in relation to relative movements at right angles to the loading direction (F).

10. Bending-moment-free clamping apparatus according to claim 8 or 9, characterized in that, given a conical construction of the facets (3, 3′), the balls (2, 2′) at the sample ends are inserted into the spherical shells (4, 4′) in such a way that the cone angles extend in opposite directions to one another.

## Revendications

1. Dispositif de fixation sans moment de flexion, pour éprouvettes ou échantillons, en particulier échantillons de céramique, pour soumettre l'éprouvette à une sollicitation en traction et/ou en compression, avec des éléments récepteurs, pour recevoir avec ajustement de forme les extrémités de l'éprouvette, les éléments récepteurs présentant des faces extérieures sphériques, reçues dans des surfaces conjuguées sphériques, caractérisé en ce que les éléments récepteurs sont réalisés sous forme de sphères (2, 2′), pouvant être insérées dans des trous ou des perçages transversaux (9) adaptés, ménagés sur les extrémités d'éprouvettes.

2. Dispositif de fixation sans moment de flexion selon la revendication 1, caractérisé en ce que les sphères (2, 2′) sont réalisées sous forme de sphères en acier, en céramique, en métal dur ou en matériau composite.

3. Dispositif de fixation sans moment de flexion selon la revendication 1 ou 2, caractérisé en ce que les sphères (2,2′) sont reçues dans des coussinets sphériques (4, 4′) pouvant être appliqués sans jeu contre les sphères (2, 2′) chacun par l'intermédiaire d'une bague de maintien (6, 6′) ou analogue.

4. Dispositif de fixation sans moment de flexion selon l'une des revendications précédentes, caractérisé en ce que les coussinets sphériques (4, 4′) présentent des surfaces extérieures coniques ou cunéiformes et les bagues de maintien présentent des surfaces conjuguées ou intérieures de réalisation correspondante.

5. Dispositif de fixation sans moment de flexion selon l'une des revendications précédentes, caractérisé en ce que les sphères (2,2′) et/ou les coussinets sphériques (4, 4′) présentent un revêtement à faible coefficient de frottement.

6. Dispositif de fixation sans moment de flexion selon l'une des revendications précédentes, caractérisé en ce qu'un moyen de lubrification est introduit entre les sphères (2,2′) et les coussinets sphériques (4, 4′).

7. Dispositif de fixation sans moment de flexion selon l'une des revendications précédentes, caractérisé en ce que les sphères (2,2′) présentent chacune un méplat (3, 3′) annulaire, sur la périphérie de la sphère, pour recevoir des éprouvettes plates (1).

8. Dispositif de fixation sans moment de flexion selon la revendication 7, caractérisé en ce que les méplats (3, 3′) sont cylindriques ou coniques (tronconiques) et les trous ou les alésages transversaux (9) ménagés dans les extrémités d'éprouvettes sont chacun adaptés à la configuration des méplats (3, 3′).

9. Dispositif de fixation sans moment de flexion selon la revendication 8, caractérisé en ce qu'en cas de réalisation conique (tronconique) des méplats (3, 3′) la conicité, ou l'angle de cône, est choisi si petit qu'un auto-blocage se produit entre l'éprouvette (1) et la sphère (2,2′) par rapport aux mouvements relatifs orientés transversalement par rapport à la direction de sollicitation (F) sur la surface de méplat ou d'appui, en cas de sollicitation en traction ou en compression de l'éprouvette (1).

10. Dispositif de fixation sans moment de flexion selon la revendication 8 ou 9, caractérisé en ce qu'en cas de réalisation conique des méplats (3, 3′) les sphères (2,2′) sont insérées sur les extrémités d'éprouvettes, dans les coussinets sphériques (4, 4′), de manière que les angles de cônes soient orientés en direction inverse les uns des autres.
